# EUROPÄISCHE PATENTSCHRIFT

(11) **EP 3 678 589 B1**
(45) Veröffentlichungstag und Bekanntmachung des Hinweises auf die Patenterteilung: **19.02.2025**
(21) Anmeldenummer: 18773947.9
(22) Anmeldetag: 06.09.2018
(51) Int. Cl.: A61F 2/07, A61F 2/06, A61F 2/89, A61F 2/852

(54) **INTRALUMINALES GEFÄSSPROTHESENSYSTEM**
INTRALUMINAL VESSEL PROSTHESIS SYSTEM
SYSTÈME DE PROTHÈSE VASCULAIRE INTRALUMINAL

(30) Priorität: 08.09.2017 DE 102017120819
(43) Veröffentlichungstag der Anmeldung: 15.07.2020
(73) Patentinhaber: JOTEC GmbH, 72379 Hechingen (DE)
(72) Erfinder: YOUSSEF, Marwan, 55122 Mainz (DE); WOERNE, Christian, 73760 Ostfildern (DE); WALTHER, Michael, 24632 Lentfoehrden (DE)
(74) Vertreter: Witte, Weller & Partner Patentanwälte mbB
(86) Internationale Anmeldenummer: PCT/EP2018/074019
(87) Internationale Veröffentlichungsnummer: WO 2019/048551

(56) Entgegenhaltungen:
- EP-A1- 2 740 441
- WO-A1-2013/025727
- WO-A1-2013/155306
- WO-A1-2017/114879
- US-A1- 2017 007 392

## Beschreibung

Die vorliegende Erfindung betrifft eine intraluminale Gefäßprothese und ein intraluminales Gefäßprothesensystem zur Implantation im Bereich des Aortenbogens eines Patienten.

Derartige Gefäßimplantate sind im Stand der Technik bspw. aus der DE 103 37 739.5 bekannt.

Allgemein ist es bekannt, intraluminale Gefäßprothesen, die auch als endovaskuläre Gefäßstents/-stentgrafts bezeichnet werden, zur Behandlung von geschwächten, verletzten, gerissenen oder aneurysmatischen Gefäßen einzusetzen. Hierfür wird an der erkrankten oder verletzten Stelle des Gefäßes eine Gefäßprothese bzw. ein Stentgraft freigesetzt, die/der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Die zur Behandlung derartiger Aneurysmen verwendeten selbstexpandierenden Gefäßimplantate bestehen im Allgemeinen aus einem hohlzylindrischen Metallrahmen bzw.-gerüst, dessen Mantelfläche mit einer Textil- oder Polymerfolie abgedeckt ist, so dass sich ein hohlzylindrischer Körper ergibt. Zur Implantation wird die Gefäßprothese radial zusammengedrückt, so dass sich seine Querschnittsfläche deutlich verringert. Die Gefäßprothese wird dann mit Hilfe eines Einführsystems in den Bereich des Aneurysma gebracht, wo es freigesetzt wird. Auf Grund der Federwirkung des Metallrahmens/-gerüsts expandiert die Gefäßprothese wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch die Gefäßprothese und eine weitere Belastung der Aussackung wird verhindert.

Der Metallrahmen solcher Gefäßprothesen besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Prothesenmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Allgemein treten Aneurysmen häufig im Bereich der Baucharterie (Aorta *abdominalis*) oder Brustarterie (*Aorta thoracica*) auf. Zur Behandlung von Aneurysmen in der Bauch- oder Brustarterie ist es bereits bekannt, die Arterie durch Implantation eines Stents soweit zu stabilisieren, dass eine Ruptur des Gefäßes vermieden wird.

Aneurysmen können aber auch in dem sogenannten aufsteigenden Ast der Aorta (*Aorta ascendens*) auftreten. Der aufsteigende Ast der Aorta ist unmittelbar mit dem Herz verbunden. Ausgehend von der Aortenwurzel (*Sinus aortae*) verläuft der aufsteigende Ast in leicht gekrümmter Form vom Herz weg nach oben und geht dort in den Aortenbogen (*Arcus aortae*) über, und führt in den absteigenden Ast der Aorta (Aorta *descendens*). Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit der Brustarterie und im weiteren Verlauf mit der Baucharterie.

Nicht nur im Bereich des Aortenbogens ist es wichtig, vom Hauptgefäße abgehende Seiten-Gefäße durch die Positionierung der Gefäßprothese nicht zu blockieren, weshalb viele Gefäßprothesen offene Zonen oder sogenannte Fenestrierungen aufweisen, über welche vom Gefäßimplantat abgehende Seitenäste, die in die Seiten-Gefäße hineinragen, eingeführt und am Gefäßimplantat fixiert werden können.

Gefäßerkrankungen wie Aneurysmen oder Dissektionen im Bereich des Aortenbogens werden bis heute in der Regel in einem invasiven offenen Eingriff behandelt. Eine solche Operation macht bisher regelmäßig zwei große, zeitlich getrennte Eingriffe erforderlich, und stellt einen sehr großen und aufwändigen und damit gefährlichen Eingriff dar, da nicht nur das Herz, sondern auch das Gehirn und die Bauchorgane des Patienten einer hypothermen Perfusion, d.h. also einer künstlichen, kalten extrakorporalen Durchblutung, bzw. einem hypothermen Durchblutungsstillstand unterzogen werden müssen. Mit einem solchen Eingriff sind jedoch nur wenige Herzchirurgen an erfahrenen Zentren vertraut.

Aus der WO 2013/025727 A1 ist eine Gefäßprothese bekannt, bei der über eine in der Prothesenwandung vorgesehene Öffnung, die wiederum in einer Vertiefung in der Wandung vorgesehen ist, Gefäßprothesen-Seitenäste im Lumen der Gefäßprothese verankert werden können. Aus der WO 2013/155306 A sind ferner verzweigte, doppellumige Gefäßprothesen bekannt.

Ferner sind gegenwärtig auch Stents und Stentgrafts bzw. Kombinationen von Stents bekannt, die minimalinvasiv eingeführt werden. Diese sind jedoch bei vielen Patienten mit erkrankten Gefäßen nur bedingt einsetzbar.

Es besteht daher nach wie vor ein großes Bedürfnis nach Stent-/Stentgraftsystemen, bzw. Gefäßprothesen, die zur Implantation im Bereich des Aortenbogens und zur Behandlung von Gefäßerkrankungen im Bereich des Aortenbogens eingesetzt werden können.

Aufgabe der vorliegenden Erfindung ist es daher, eine Gefäßprothese sowie ein Gefäßprothesensystem bereitzustellen, mit welchem der Bereich der aufsteigenden Aorta, des Aortenbogens und der absteigendem Aorta einer Vielzahl verschiedener Patienten mit unterschiedlichen Gefäßbeschaffenheiten schnell und unkompliziert behandelt werden können, bzw. das die oben geschilderten Eingriffe auch weniger erfahrenen Herzchirurgen ermöglicht.

Gemäß der vorliegenden Erfindung wird diese Aufgabe gelöst durch ein intraluminales Gefäßprothesensystem zur Implantation im Bereich des Aortenbogens eines Patienten gemäß den beigefügten Ansprüchen, das zumindest folgendes aufweist: eine hohlzylindrische Haupt-Gefäßprothese, die ein durch die Haupt-Gefäßprothese geführtes Lumen, ein erstes Lumenende, ein zweites Lumenende, ein hohlzylindrisches Stentgerüst, ggf. mit einem daran befestigtes Prothesenmaterial, eine Länge L1 und einen Durchmesser D1 aufweist, wobei die hohlzylindrische Haupt-Gefäßprothese für die Implantation im Bereich des Aortenbogens und der *Aorta descendens* des Patienten ausgestaltet und dimensioniert ist, und wobei die Haupt-Gefäßprothese zumindest eine hohlzylindrische Verankerungs-Gefäßprothese aufweist, welche ein durch die Verankerungs-Gefäßprothese geführtes Lumen, ein erstes Lumenende, ein zweites Lumenende, ein hohlzylindrisches Stentgerüst, ggf. mit einem daran befestigtes Prothesenmaterial, eine Länge L2 und einen Durchmesser D2 aufweist, wobei die Verankerungs-Gefäßprothese innerhalb des Lumens der Haupt-Gefäßprothese zumindest über einen Teil der Länge L2 der Verankerungs-Gefäßprothese fest angebracht ist, und wobei der Durchmesser D2 der Verankerungs-Gefäßprothese um mindestens 45% kleiner ist als der Durchmesser D1 der Haupt-Gefäßprothese, und wobei die Länge L2 der Verankerungs-Gefäßprothese kleiner ist als die Länge L1 der Haupt-Gefäßprothese.

Das erfindungsgemäße intraluminale Gefäßprothesensystem zur Implantation im Bereich des Aortenbogens umfasst ferner zumindest eine hohlzylindrische Seiten-Gefäßprothese, ein durch die Seiten-Gefäßprothese geführtes Lumen die ein erstes Lumenende, ein zweites Lumenende, ein hohlzylindrisches Stentgerüst, ggf. mit einem daran befestigtes Prothesenmaterial, eine Länge L3 und einen Durchmesser D3 aufweist, wobei die hohlzylindrische Seiten-Gefäßprothese für die Implantation zur Überbrückung des Abgangs der *Arteria subclavia* oder der *Arteria carotis* des Patienten ausgestaltet und dimensioniert ist, derart, dass die Seiten-Gefäßprothese mit ihrem ersten Lumenende in der *Arteria subclavia* oder der *Arteria carotis* positionierbar ist, und mit ihrem zweiten Lumenende zur sicheren Verankerung der Seiten-Gefäßprothese über das erste Lumenende der Verankerungs-Gefäßprothese zumindest teilweise in deren Lumen einführbar und darin fixierbar ist.

Offenbart hierin ist ferner ein Verfahren zur Implantation eines intraluminalen Gefäßprothesensystems in den Aortenbogen eines Patienten, umfassend die folgenden Schritte:
- Einführen und Freisetzen der Haupt-Gefäßprothese in den Bereich des Aortenbogens und der *Aorta descendens,* und
- Einführen der Seiten-Gefäßprothese und Freisetzen derselben mit ihrem ersten Lumenende in der *Arteria subclavia* oder der *Arteria carotis* und mit ihrem zweiten Lumenende über das erste Lumenende der Verankerungs-Gefäßprothese zumindest teilwiese in deren Lumen.

Die der Erfindung zugrunde liegende Aufgabe wird auf diese Weise vollkommen gelöst.

Mit der neuen Gefäßprothese bzw. dem neuen Gefäßprothesen-System wird erreicht, dass auf einfache Weise ein geschwächtes, verletztes, gerissenes oder aneurysmatisches Gefäß, insbesondere im Bereich des Aortenbogens, behandelt werden kann. Erfindungsgemäß wird damit eine Gefäßprothese bereitgestellt, mit welcher die Möglichkeit geschaffen wird, Eingriffe insbesondere am Aortenbogen, bzw. in der aufsteigenden Aorta, dem Aortenbogen und der absteigenden Aorta, chirurgisch zu vereinfachen und zeitlich deutlich zu verkürzen.

Insbesondere können durch die erfindungsgemäße Gefäßprothese bzw. das erfindungsgemäße Gefäßprothesensystem solche Gefäße behandelt werden, welche im Bereich der zu behandelnden Stelle abgehende Seitengefäße aufweisen. Durch den besonderen Aufbau können die Seitengefäße über zumindest eine Seiten-Gefäßprothese weiterhin mit Blut versorgt werden, wobei gleichzeitig die verletzten betroffenen Gefäße durch die intraluminale Gefäßprothese gestützt werden. Die Einführung und Platzierung dieses Systems kann durch dessen zumindest zweiteiligen Aufbau, aus hohlzylindrischer Hauptgefäßprothese zusammen mit der Verankerungs-Gefäßprothese und aus der Seiten-Gefäßprothese, präzise und einfach gehandhabt werden.

Zur Einführung des Gefäßprothesensystems in ein Gefäß eines zu behandelnden Patienten wird zunächst die intraluminale Gefäßprothese an der gewünschten Stelle im Gefäß implantiert, bevorzugt hierbei ist der Aortenbogen. Anschließend wird eine Seiten-Gefäßprothese in das Seitengefäß implantiert, wobei das erste Lumenende im Seitengefäß positioniert wird während das zweite Lumenende zur sicheren Verankerung der Seiten-Gefäßprothese über das erste Lumenende der Verankerungs-Gefäßprothese zumindest teilweise in deren Lumen einführbar und darin fixierbar ist.

Aufgrund des besonderen Aufbaus können die Gefäßprothese und die Seiten-Gefäßprothese getrennt voneinander implantiert werden. Die erfindungsgemäße Gefäßprothese sowie das erfindungsgemäße Gefäßprothesensystem bieten daher den Vorteil, dass nicht nur hoch spezialisierte Herzchirurgen die weiter oben geschilderten Eingriffe am Aortenbogen vornehmen können, sondern auch unerfahrene Fachkräfte. Ferner bietet die Erfindung den Vorteil, dass die Gefäßprothese sowie das Gefäßprothesensystem den jeweiligen anatomischen Verhältnissen des zu behandelnden Patienten angeglichen werden kann.

Aufgrund des Aufbaus des erfindungsgemäßen Gefäßprothesensystems, bei dem die Seiten-Gefäßprothese in die Verankerungs-Gefäßprothese zumindest teilweise verankert bzw. "gesteckt" wird, können anatomisch sehr unterschiedliche Gefäße mit dem gleichen Gefäßprothesensystem behandelt werden. Dies kann erreicht werden durch die unterschiedliche Einsteck-/Verankerungstiefe der Seiten-Gefäßprothese in die Verankerungs-Gefäßprothese. Liegen die Seitengefäße weiter weg von der eigentlichen Haupt-Gefäßprothese, so kann die Einstecktiefe geringer ausgewählt werden, im Gegensatz zu einer größeren Verankerungstiefe bei nahgelegenen Seitengefäßen.

Die Implantation des Gefäßprothesensystems ist insbesondere deshalb vereinfacht möglich, da die Haupt-Gefäßprothese nicht genau, in Bezug zu den abgehenden Seitengefäßen, ausgerichtet werden muss. Korrigiert werden kann die Ausrichtung durch das erfindungsgemäße "Verankerungssystem" und der damit verbundenen jeweiligen Einsteck-/Verankerungstiefe der Seiten-Gefäßprothese in die Verankerungs-Gefäßprothese. Gerade die Ausrichtung einer aus dem Stand der Technik bekannten einteiligen Gefäßprothese mit abgehenden Seitengefäßen erschwert nicht nur die Implantation, sondern auch die Fertigung solcher Implantate.

Erfindungsgemäß kann sowohl die Haupt-Gefäßprothese, die zumindest eine Verankerungs-Gefäßprothese als auch die zumindest eine Seiten-Gefäßprothese als gecoverter oder ungecoverter Stent vorliegen; also gegebenenfalls mit einem Prothesenmaterial zumindest teilweise ummantelt sein, bzw. ein an dem Satengerüst befestigtes Prothesenmaterial aufweisen. Je nach Verletzung im Gefäß und den anatomischen Gegebenheiten kann es sinnvoll sein, dass zumindest eine der aufgeführten Gefäßprothesenteile kein Prothesenmaterial aufweist; beispielsweise die Seiten-Gefäßprothese oder die Verankerungs-Gefäßprothese.

Insbesondere ist gemäß einer Ausführungsform bevorzugt, wenn die Haupt-Gefäßprothese, sofern sie ein Prothesenmaterial aufweist, einen Fenestrierungsbereich aufweist, also einen Bereich, über welchen die Seiten-Gefäßprothese in das Lumen der im Lumen der Haupt-Gefäßprothese vorliegenden Verankerungs-Gefäßprothese zumindest teilweise eingeführt und verankert werden kann. Dieser Fenestrierungsbereich kann bspw. ausgewählt sein aus Fenestrierungsbereichen im Prothesenmaterial, einzelnen Öffnungen im Mantelbereich der Haupt-Gefäßprothese, oder einen offenen Bereich, der sich umlaufend über den gesamten Umfang der Haupt-Gefäßprothese in diesem Bereich erstreckt.

Alternativ kann die Seiten-Gefäßprothese über das erste Lumenende der Haupt-Gefäßprothese in das Lumen der im Lumen der Haupt-Gefäßprothese vorliegenden Verankerungs-Gefäßprothese zumindest teilweise eingeführt und verankert werden.

Die Haupt-Gefäßprothese kann ferner gemäß einer Ausführungsform zumindest zwei Stentgerüst-Abschnitte aufweisen bzw. aus zumindest zwei Stentgerüst-Abschnitten bestehen, die verbindbar miteinander sind, bspw. durch teilweises Einführen des zweiten Stentgerüst-Abschnitt in den zweiten Stentgerüst-Abschnitt.

Gemäß einer Ausführungsform der erfindungsgemäßen Gefäßprothese weist diese mehrere Verankerungs-Gefäßprothesen auf. Die Anzahl der Verankerungs-Gefäßprothesen richtet sich primär an der Anzahl der abgehenden Seitengefäße, welche durch die Seiten-Gefäßprothesen behandelt werden sollen.

Gemäß einer Ausführungsform der vorliegenden Erfindung sind das erste Lumenende der Haupt-Gefäßprothese und das erste Lumenende der Verankerungs-Gefäßprothese bündig angeordnet.

Dabei bedeutet "bündig angeordnet", dass die Verankerungs-Gefäßprothese mit ihrem ersten Lumenende am ersten Lumenende der Haupt-Gefäßprothese ausgerichtet ist und damit beide Lumenenden sozusagen auf gleicher Ebene enden. Die Verankerungs-Gefäßprothese erstreckt sich bei dieser Ausfürhungsform damit vom ersten Lumenende der Haupt-Gefäßprothese im Lumen der Haupt-Gefäßprothese in Richtung des zweiten Lumenendes entlang der Innenwand der Haupt-Gefäßprothese. Der Zugang zur Verankerungs-Gefäßprothese für eine Seitengefäßprothese findet damit nicht über eine Seitenwand oder eine Fenestrierung in der Haupt-Gefäßprothese statt, sondern über das erste Lumenende der Hauptgefäßprothese.

Nachstehend werden einige hierin verwendeten Begriffe, die zwar für den Fachmann an sich und unter Heranziehung der vorliegenden Offenbarung klar sind, näher definiert:

Grundsätzlich werden bei Stentgrafts oder endoluminalen Prothesen allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeuten der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. des Einführsystems, das in den Patienten eingeführt wird, bzw. das vom Anwender ausgesehen am Entferntesten liegt, und der Begriff "proximal" das Ende, das sich dem Anwender näher zugewandt ist.

Entsprechend sind vorliegend die "proximale" und die "distale" Öffnung des Gefäßimplantats die Öffnungen, durch die hindurch der Blutfluss durch das hohlzylindrische Lumen der Gefäßimplantat gewährleistet wird: Wenn das erfindungsgemäße Gefäßimplantat in einem Blutgefäß wie beispielsweise der Aorta implantiert ist, fließt also das vom Herzen kommende Blut durch die proximale Öffnung des Gefäßimplantats, und verlässt das Gefäßimplantat durch deren distale Öffnungen. Die hohlzylindrische Haupt-Gefäßprothese bzw. dessen Verankerungs-Gefäßprothese, als auch die Seiten-Gefäßprothese können dabei über ihre Gesamtlänge hinweg einen einheitlichen Durchmesser, oder aber unterschiedliche Durchmesser aufweisen.

Bei dem hohlzylindrische Stentgerüst handelt es sich erfindungsgemäß um einen Metallrahmen, der in der Regel bspw. aus einem Drahtgeflecht oder aus hintereinander angeordneten, mäanderförmig umlaufenden, sogenannten Stentfedern, die ggf. über Verbindungsstützen aus Draht miteinader verbunden sind, oder die lediglich über das Prothesenmaterial miteinander verbunden sind. Das Drahtgeflecht bzw. die Stentfedern sind üblicherweise aus deinem Shape-Memory-Material, in der Regel aus Nitinol, wodurch die Stentfedern nach der Implantation in ein Gefäß zur Freisetzung wieder in den expandierten Zustand übergehen und die Gefäßprothese dadurch "aufspannt".

Gemäß einer ersten Ausführungsform des erfindungsgemäßen Gefäßprothesensystems weist/weisen die Haupt-Gefäßprothese und/oder die Verankerungs-Gefäßprothese und/oder die Seiten-Gefäßprothese ein Stentgerüst sowie ein daran befestigtes Prothesenmaterial auf.

Dabei bedeutet vorliegend "Stentgerüst" jedes Metallgerüst, das durch ein Aufspannen oder durch eine Expansion des Gerüsts einer Gefäßprothese eine Expansionskraft zur Offenhaltung eines Gefäßes verleiht, ggf. in Kombination mit einem an dem Gerüst befestigten Prothesenmaterial. Das Prothesenmaterial besteht bevorzugt aus einem bioverträglichen Material, was dazu führt, dass der Kontakt zwischen dem Gefäßimplantat und der Gefäßwand komplikationsfrei ist.

In diesem Zusammenhang ist bevorzugt, wenn das Prothesenmaterial ein Material aufweist, das ausgewählt ist aus einem Textil oder einem Polymer. Insbesondere ist bevorzugt, wenn das Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

Gemäß einer weiteren Ausführungsform des erfindungsgemäßen Gefäßprothesensystems ist das Stentgerüst der Haupt-Gefäßprothese und/oder das Stentgerüst der Verankerungs-Gefäßprothese und/oder das Stentgerüst der Seiten-Gefäßprothese ausgewählt aus einem laser-geschnittenen Stentgerüst, einzelnen Stentfedern oder einem geflochtenen Stentgerüst.

Dabei wird unter "Stentgerüst" jede Ausbildung eines Stents verstanden, bei der verschieden Draht-Stränge derart miteinander verflochten, ineinander verschlungen oder anderweitig verknüpft sind, dass sich eine Struktur mit Zonen, Bereichen oder Punkten bildet, an denen die Stränge übereinander liegen, und mit Zonen oder Bereichen, die frei von den Drahtsträngen sind und daher Öffnungen oder Fenster oder Maschen bilden. Entsprechend weist auch ein Laser-geschnittenes Stent-Stützgerüst Maschen oder Öffnungen auf. Vorzugsweise sind die Öffnungen, Maschen oder Fenster hierbei diamantförmig.

Vorliegend wird unter einer "Stentfeder" jedes einstückige, ringförmige Element verstanden, das sich aufgrund dessen Materials komprimieren lässt, und sich nach Entfernung des Kompressionsdrucks wieder federartig expandieren kann.

Gemäß einer Ausführungsform weist das Stentgerüst der Haupt-Gefäßprothese und/oder das Stentgerüst der Verankerungs-Gefäßprothese und/oder das Stentgerüst der Seiten-Gefäßprothese hintereinander angeordnete, nicht miteinander verbundene mäanderförmige umlaufende Stentringe sowie ein mit den Stentringen fest verbundenes Prothesenmaterial auf.

Dabei wird vorliegend unter einem "Stentring" jedes einstückige, ringförmige Element verstanden, das durch einen umlaufenden Draht gebildet, und einen im Wesentlichen runden Umfang besitzt. Der Draht des Stentrings kann dabei in Wellen verlaufen, wobei sich Wellenberg und Wellental, die eine Phase oder Amplitude bilden, abwechseln. Dabei wird unter "mäanderförmig" vorliegend jeder schlingen- bzw. schleifenförmige Verlauf" des Stentrings bzw. des Stentdrahtes verstanden.

Das gegebenenfalls oder zwingend (bei den Stentringen) einzusetzende bzw. vorliegende Prothesenmaterial der im erfindungsgemäßen System einzusetzenden Gefäßprothesen weist dabei vorzugswiese ein Material auf, das ausgewählt ist aus einem Textil oder einem Polymer.

Dabei können das Prothesenmaterial der Haupt-Gefäßprothese und/oder der Verankerungs-Gefäßprothese und/oder der Seiten-Gefäßprothese, sofern diese jeweils oder auch nur eines oder zwei davon ein Prothesenmaterial aufweisen, aus einem solchen Material gebildet sein, oder ein solches aufweisen.

Gemäß einer weiteren Ausführungsform weist der mäaderförmgie Umlauf von zumindest einem Stentring des Stentgerüsts eine ungleichmäßige Amplitude auf.

Gemäß dieser Ausführungsform wird ein Gefäßprothesensystem bereitgestellt, welches insbesondere in gekrümmten Gefäßen, bspw. dem Aortenbogen, eingesetzt werden können, da es durch die ungleichmäßigen Amplituden nicht zu unnötiger Materialanhäufung in der Gefäßprothese im Bereich der Krümmung kommt.

Gemäß einer Ausführungsform ist das Stentgerüst der Haupt-Gefäßprothese und/oder das Stentgerüst der Verankerungs-Gefäßprothese und/oder das Stentgerüst der Seiten-Gefäßprothese selbstexpandierbar.

Dies bedeutet, dass das Stentgerüst von einem komprimierten Zustand in einen expandierten Zustand überführbar ist, wobei der komprimierte Zustand durch ein das Stentgerüst - sowie ggf. das daran befestigte Prothesenmaterial - komprimierendes und rückziehbares Element, bspw. eine Hülle, erreicht wird, die sich zur Einführung in ein Gefäß über dem Stentgerüst zu dessen Komprimierung befindet. Nach korrekter Platzierung wird das das Stentgerüst komprimierende Element entfernt, woraufhin das Stentgerüst expandiert und sich dessen Wände sich an die Gefäßwand drücken, wodurch die Gefäßprothese im Gefäß fixiert wird.

Allgemein ist bevorzugt, wenn das Stentgerüst aus einem selbstexpandierenden Material besteht oder ein solches aufweist. Hierbei ist insbesondere bevorzugt, wenn das Material Nitinol ist.

Gemäß einer Ausführungsform ist der Durchmesser D1 der Haupt-Gefäßprothese von 24 mm bis 42 mm, insbesondere 24 mm, 26 mm, 28 mm, 30 mm, 32 mm, 34 mm, 36 mm, 38 mm, 40 mm oder 42 mm.

Gemäß einer weiteren Ausführungsform ist der Durchmesser D2 der Verankerung-Gefäßprothese von 6 mm bis 14 mm, insbesondere 6 mm, 7 mm, 8 mm, 9 mm, 10 mm, 11 mm, 12 mm, 13 mm oder 14 mm.

Gemäß einer weiteren Ausführungsform ist der Durchmesser D3 der Seiten-Gefäßprothese von 6 mm bis 16 mm, insbesondere 6 mm, 7 mm, 8 mm, 9 mm, 10 mm, 11 mm, 12 mm, 13 mm, 14 mm, 15 mm oder 16 mm.

Gemäß diesen Ausführungsformen kann der Durchmesser D1, D2 und D3 an das jeweilige zu behandelnde Gefäß angepasst werden. Die genannten Maße entsprechen dabei insbesondere den Durchmessern innerhalb des Aortenbogens bzw. der *Arteria Truncus brachiocephalicus,* der *Arteria carotos communis* und der *Arterie subclavia sinistra.* Die Haupt-Gefäßprothese, die Verankerung-Gefäßprothese sowie die Seiten-Gefäßprothese kann erfindungsgemäß über die gesamte Länge hinweg einen einheitlichen Durchmesser, oder aber unterschiedliche Durchmesser aufweisen. Sofern die jeweiligen Gefäßprothesenteile einen unterschiedlichen Durchmesser aufweisen, so entsprechen die genannten Durchmesser von D1, D2 und D3 dieser Ausführungsformen den größten bzw. maximalen Durchmessern.

Gemäß einer weiteren Ausführungsform ist die Verankerungs-Gefäßprothese im Lumen der Haupt-Gefäßprothese an einer Innenwand angebracht, vorzugsweise angenäht, angeklebt, oder angeschweißt.

Eine sichere Befestigung der Verankerungs-Gefäßprothese im Lumen der Haupt-Gefäßprothese ist bevorzugt, da sich diese ansonsten ungewollt von der Haup-Gefäßprothese lösen kann. Dies kann durch die Ausführungsform gewährleistet werden. Die Verankerungs-Gefäßprothese ist aufgrund ihres Aufbaus nicht selbst im Gefäß verankert, wie bspw. die Haupt-Gefäßprothese bzw. die Seiten-Gefäßprothese. Aufgrund eines starken Blutflusses innerhalb der Gefäßprothese kann sich demnach die Verankerungs-Gefäßprothese gegebenenfalls lösen und sich ungewollt verschieben. Dieser Nachteil wird durch die beschriebene Ausführungsform vermieden.

Erfindungsgemäß ist das zweite Lumenende der Seiten-Gefäßprothese über das erste Lumenende der Haupt-Gefäßprothese in das erste Lumenende der Verankerungs-Gefäßprothese und zumindest teilweise in deren Lumen einführbar.

Damit ist es vereinfacht möglich Seitengefäße mit einer Seiten-Gefäßprothese zu versehen. Mittels des Verankerungssystems der Seiten-Gefäßprothese in die Verankerungs-Gefäßprothese ist es auch einem unerfahrenen Herzchirurgen möglich derartige Implantationen durchführen zu können, da die Haupt-Gefäßprothese nicht notwendigerweise genau auf die Seitengefäße ausgerichtet sein muss. Ein Fehler innerhalb der Implantation betreffend die Lokation der Haupt-Gefäßprothese kann durch das Ineinanderstecken ausgeglichen werden.

Gemäß einer weiteren Ausführungsform weist die Haupt-Gefäßprothese eine Mantelfläche sowie eine in der Mantelfläche vorgesehene Fenestrierung auf, über welche das zweite Lumenende der Seiten-Gefäßprothese ins Lumen der Haupt-Gefäßprothese und über das erste Lumenende der Verankerungs-Gefäßprothese in deren Lumen zumindest teilweise einführbar ist.

Unter fenestrierten Gefäßprothesen werden solche verstanden, die präformierte Löcher (Fenestrationen) aufweisen, um ein oder mehrere Gefäßabgänge in der Gefäßprothese zu ermöglichen. Gemäß dieser Ausführungsform ist es möglich, seitlich abgehende Blutgefäße über eine Seiten-Gefäßprothese weiter mit Blut zu versorgen und gleichzeitig das Hauptgefäß durch eine Haupt-Gefäßprothese zu stützen.

Alternativ kann die Fenestrierung als *in-situ* Fenestrierung ausgestaltet sein. Hierbei wird die Fenestrierung erst eingebracht, nachdem die Gefäßprothese im Gefäß positioniert wurde. Hier wird die Haupt-Hauptgefäßprothese in situ mit einer Nadel penetriert, um ein Nadelloch in der Mantelfläche des Prothesenmaterials zu bilden. Anschließend wird eine Dilator-Anordnung durch das Nadelloch geschoben, um das Nadelloch zu erweitern.

Die Mantelfläche ist bevorzugt aus einem Prothesenmaterial ausgestaltet, welches mit dem Stentgerüst der Haupt-Gefäßprothese verbunden ist.

Das erfindungsgemäße intraluminale Gefäßprothesensystem kann zur Behandlung von Gefäßerkrankungen eines Patienten, insbesondere im Bereich des Aortenbogens und der *Aorta descendens* verwendet werden.

In dem hierin offenbarten Verfahren kann die Seiten-Gefäßprothese über das erste Lumenende der Haupt-Gefäßprothese in das Lumen der Verankerungs-Gefäßprothese eingeführt werden.

In dem hierin offenbarten Verfahren kann die Haupt-Gefäßprothese ein Mantelfläche sowie eine in der Mantelfläche vorgesehene Fenestrierung aufweisen, und die Seiten-Gefäßprothese kann über die Fenestrierung in der Haupt-Gefäßprothese ins Lumen der Haupt-Gefäßprothese und über das erste Lumenende der Verankerungs-Gefäßprothese in deren Lumen zumindest teilweise eingeführt werden.

Die für die erfindungsgemäße Gefäßprothese bzw. das erfindungsgemäße Gefäßprothesensystem beschriebenen Merkmale, Eigenschaften und Vorteile können für die hierin offenbarten Verfahren entsprechend angewandt werden.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Fig. 1 eine schematische Darstellung einer ersten Ausführungsform einer Haupt-Gefäßprothese einer erfindungsgemäßen intraluminalen Gefäßprothese;
Fig. 2 eine schematische Darstellung der Ausführungsform aus Fig. 1, wobei die intraluminale Haupt-Gefäßprothese im Aortenbogen bzw. der *Aorta descendens* implantiert vorliegt;
Fig. 3a eine schematische Darstellung einer ersten Ausführungsform des erfindungsgemäßen intraluminalen Gefäßprothesensystem, wobei das intraluminale Gefäßprothesensystem im Aortenbogen bzw. der *Aorta descendens* implantiert vorliegt;
Fig. 3b eine schematische Darstellung einer ersten Ausführungsform der hohlzylindrischen Seiten-Gefäßprothese; und
Fig. 4 eine schematische Darstellung einer zweiten Ausführungsform des erfindungsgemäßen intraluminalen Gefäßprothesensystem, wobei das intraluminale Gefäßprothesensystem im Aortenbogen bzw. der *Aorta descendens* implantiert vorliegt.

In den Figuren werden gleiche Merkmale mit gleichen Bezugszeichen versehen, wobei aus Gründen der Übersichtlichkeit nicht in allen Figuren immer sämtliche Bezugszeichen angegeben sind.

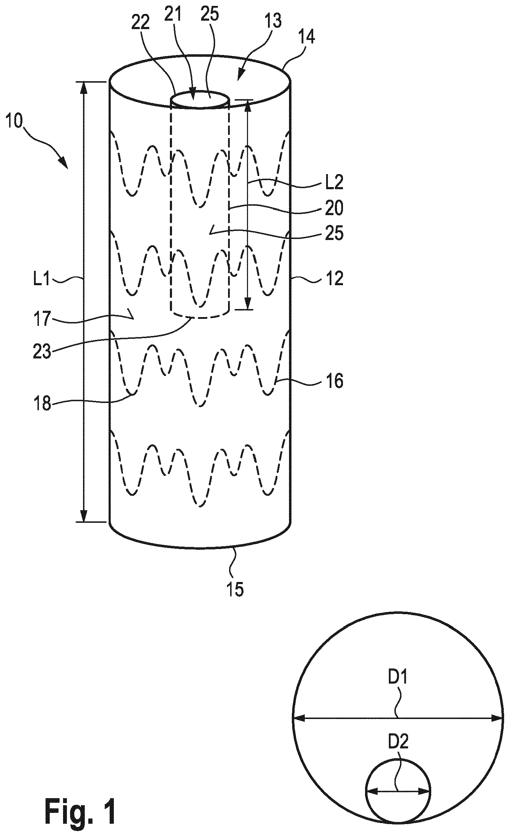

Fig. 1 zeigt eine schematische Darstellung von Teilen einer ersten Ausführungsform eines erfindungsgemäßen intraluminalen Gefäßprothesensystems. Das hier gezeigte Gefäßprothesensystem 10 weist zwei separate Gefäßprothesen auf, die miteinander fest verbunden sind; zum einen die Haupt-Gefäßprothese 12 und zum anderen die Verankerungs-Gefäßprothese 20. Die Befestigung der Verankerungs-Gefäßprothese 20 an der Haupt-Gefäßprothese 12 kann beispielsweise durch Annähen, Ankleben oder Anschweißen erreicht werden. Sowohl die Haupt-Gefäßprothese 12, als auch die Verankerungs-Gefäßprothese 20 weisen jeweils ein Lumen 13 und 21 auf. Dieses erstreckt sich jeweils vom ersten Lumenende 14 und 22 bis zum zweiten Lumenende 15 und 23, sodass eine Länge L1 und L2 gebildet wird. Die hohlzylindrische Struktur der Hauptgefäßprothese 12 bzw. der Verankerungs-Gefäßprothese 20, mit dem Durchmesser D1 und D2, wird insbesondere erreicht durch das jeweilige hohlzylindrische Stentgerüst 16 und 24 und das jeweilig umgebende Prothesenmaterial 17 und 25. In dieser Darstellung ist das Stentgerüst 16 und 24 jeweils aus einzelnen Stentringen 18 und 26 aufgebaut, die nur über das Prothesenmaterial 17 und 18 miteinander verbunden sind.

Die Dimension der hohlzylindrischen Haupt-Gefäßprothese 12 entspricht in etwa der Dimension des Aortenbogens und der *Aorta decendens.* Dies ermöglicht eine einfache Implantation in das Gefäß. Je nach Beschaffenheit des Gefäßes der einzelnen Patienten können die Dimensionen des intraluminalen Gefäßprothesensystems angepasst werden. Insbesondere ist der Durchmesser D1 der Haupt-Gefäßprothese 12 an den Durchmesser der *Aorta descendens* angepasst, derart, dass diese im expandierten Zustand an die Gefäßwand gedrückt wird. Unter Umständen ist es bevorzugt, wenn die Haupt-Gefäßprothese 12 und/oder die Verankerungs-Gefäßprothese 20 über die Länge L1 bzw. L2 einen unterschiedlichen Durchmesser D1 bzw. D2 aufweisen.

Die Verankerungs-Gefäßprothese 20 ist zumindest über einen Teil der Länge L2 innerhalb des Lumens 13 der Haupt-Gefäßprothese fest angebracht, wobei der Durchmesser D2 der Verankerungs-Gefäßprothese 20 um mindestens 45% kleiner ist als der Durchmesser D1 der Haupt-Gefäßprothese 12. Zudem ist die Länge L2 der Verankerungs-Gefäßprothese 20 kleiner als die Länge L1 der Haupt-Gefäßprothese 12.

Dieser Aufbau des erfindungsgemäßen Gefäßprothesensystems ermöglicht es, im Bereich der aufsteigenden Aorta, des Aortenbogens und der absteigendem Aorta eine Vielzahl an Patienten mit unterschiedlichen Gefäßbeschaffenheiten behandeln zu können.

Die innenliegende hohlzylindrische Verankerungs-Gefäßprothese 20 dient insbesondere dem Zweck, dass zumindest teilweise innerhalb des Lumens 21 eine weitere hohlzylindrische Seiten-Gefäßprothese 30 mit ihrem zweiten Lumenende in diesem einführbar und darin fixierbar ist (in dieser Figur nicht gezeigt).

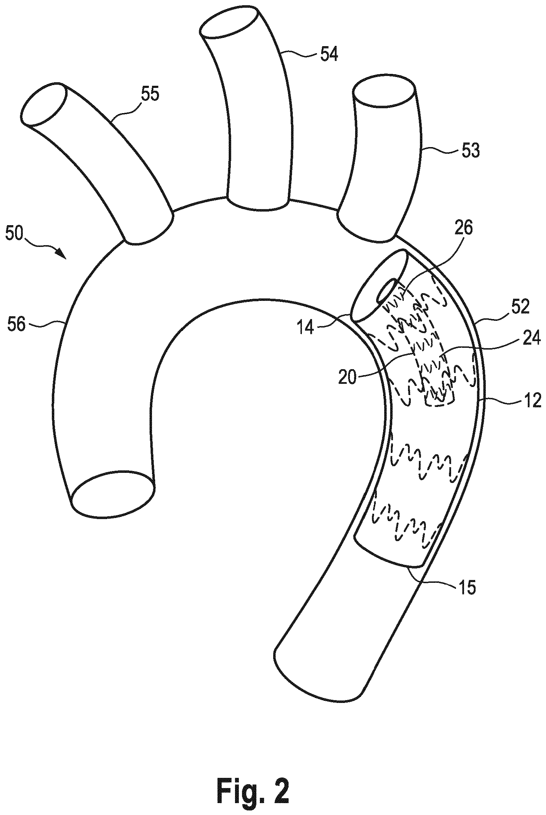

In Fig. 2 ist eine schematische Darstellung der ersten Ausführungsform des erfindungsgemäßen intraluminalen Gefäßprothesensystems gezeigt, wobei die intraluminale Haupt-Gefäßprothese 12 im Aortenbogen 50 bzw. im Bereich der Aorta *descendens* 52 implantiert vorliegt.

Der aufsteigende Ast, auch *Aorta ascendens* 56 genannt ist über die in Fig. 2 nicht gezeigte Aortenwurzel (*Sinus aorta*) mit der ebenfalls nicht dargestellten linken Kammer des Herzens verbunden. Die *Aorta ascendens* 56 ist über den Aortenbogen 50 mit der *Aorta descendens* 52 verbunden. Im Bereich des Aortenbogens 50 gehen arterielle Kopfgefäße ab, nämlich der Arterienstamm *Truncus brachiocephalicus* 55, die *Arteria carotos communis* 54 und die *Arteria subclavia sinistra* 53.

Die Haupt-Gefäßprothese 12 des intraluminalen Gefäßprothesensystems 10 überbrückt in der gezeigten Darstellung ein Aneurysma im Bereich der *Aorta descendens* 52. Der aus der *Aorta ascendens* 56 kommende Blutstrom gelangt über den Aortenbogen 50 in das erste Lumenende 14 der Haupt-Gefäßprothese 10 und verlässt diesen am zweiten Lumenende 15. Zu diesem Zweck weist die Haupt-Gefäßprothese 10 einen hohlzylindrischen Körper auf welcher ein erstes Lumen 13 formt. Dieses Lumen 13 ist gebildet durch mäanderförmige Stentringe 18, die insgesamt das Stentgerüst 16 bilden. Verbunden sind die einzelnen Stentringe 18 durch ein Prothesenmaterial 17. Das Prothesenmaterial 17 ist bevorzugt aus einem textilen Material oder eine Folie und ist durch Nähen, Kleben oder Einschmelzen an den Stentringen 18 befestigt.

Die Verankerungs-Gefäßprothese 20 kann mit einem Prothesenmaterial 25 umgeben sein. Sofern diese kein Prothesenmaterial 25 aufweist, so sind die einzelnen Stentringe 26 derart ausgebildet, dass diese ein zusammenhängendes Stentgerüst 24 bilden.

Die Haupt-Gefäßprothese 10 ist bevorzugt derart im Gefäß implantiert, dass eine Seiten-Gefäßprothese 30 möglichst platzsparend in das Lumen 21 der Verankerungs-Gefäßprothese 20 eingeführt werden kann. Das bedeutet, dass die Seiten-Gefäßprothese 30 derart mit der Verankerung-Gefäßprothese 20 verbunden wird, dass es nicht zu einer Materialanhäufung kommt.

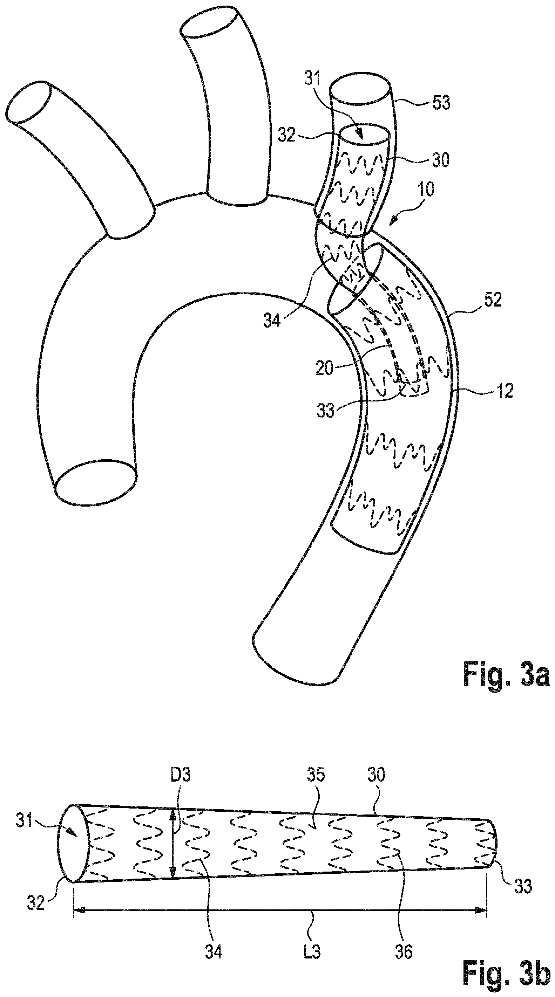

In Fig. 3a ist eine schematische Darstellung einer ersten Ausführungsform des erfindungsgemäßen intraluminalen Gefäßprothesensystems 10 gezeigt, wobei das intraluminale Gefäßprothesensystem 10 im Aortenbogen 50 bzw. der *Aorta descendens* 52 implantiert vorliegt. Das intraluminalen Gefäßprothesensystem 10 weist eine Haupt-Gefäßprothese 10 sowie eine Seiten-Gefäßprothese 30 auf. Die Haupt-Gefäßprothese 10 entspricht dabei der bereits in Fig.2 beschriebenen Haupt-Gefäßprothese 10.

Die Seiten-Gefäßprothese 30 mit einer Länge L3 weist ein durchgängiges Lumen 31 auf, welches sich vom ersten Lumenende 32 bis zum zweiten Lumenende 33 erstreckt. Das Lumen 31 wird insbesondere aus dem hohlzylindrischen Stentgerüst 34 gebildet, welches wiederrum aus einzelnen Stentringen 36 ausgebildet ist. In dieser Darstellung sind die einzelnen Stentringe 36 untereinander verbunden und nicht mit einem Prothesenmaterial umgeben. In dieser Ausgestaltung kann das Blut, kommend vom Aortenbogen, durch das Stentgerüst 34 hindurch in das abgehende Seitengefäß 53 fließen. Gleichzeitig ist das Seitengefäß 53 gestützt durch die Seiten-Gefäßprothese 30.

In einer nicht dargestellten Ausführungsform kann die Seiten-Gefäßprothese 30 auch nur teilweise durch ein Prothesenmaterial 35 umgeben sein; bevorzugt im Bereich des zweiten Lumenendes 33.

In Fig. 3a ist eine schematische Darstellung einer ersten Ausführungsform der hohlzylindrischen Seiten-Gefäßprothese 30 gezeigt. Die Seiten-Gefäßprothese 30 weist ein Lumen 31 auf, welches sich vom ersten Lumenende 32 bis hin zum zweiten Lumenende 33 erstreckt. Hierdurch definiert sich auch die Länge L3 der Seiten-Gefäßprothese 30. In dieser Darstellung weist die Seiten-Gefäßprothese 30 Stentringe 36 auf, welche untereinander mit einem Prothesenmaterial 35 verbunden sind. Das Prothesenmaterial 35 sowie die einzelnen Stentringe 36 bilden zusammen das hohlzylindrische Stentgerüst 36.

In dieser Ausgestaltung verjüngt sich die Seiten-Gefäßprothese 30 zum zweiten Lumenende 33 hin. Der Durchmesser D3 entspricht in dieser Darstellung dem größeren Durchmesser am ersten Lumenende 32. In dieser Ausgestaltung entspricht der Durchmesser am ersten Lumenende 32 dem Durchmesser des Seitengefäßes, in welches die Seiten-Gefäßprothese 30 implantiert werden soll, und der Durchmesser am zweiten Lumenende 33 dem Durchmesser der Verankerungs-Gefäßprothese 20, in welche die Seiten-Gefäßprothese 30 eingeführt werden soll.

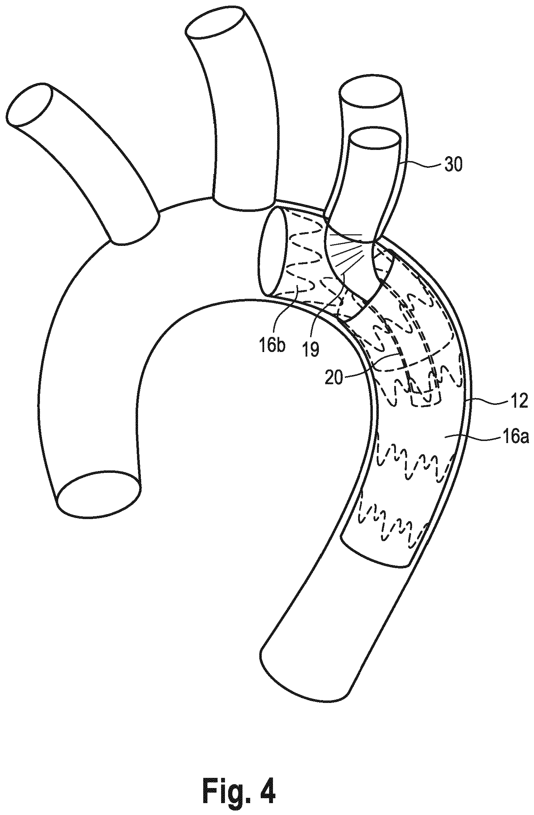

In Fig. 4 ist eine schematische Darstellung einer zweiten Ausführungsform des erfindungsgemäßen intraluminalen Gefäßprothesensystem 10 gezeigt, wobei das intraluminale Gefäßprothesensystem 10 im Aortenbogen 50 bzw. der *Aorta descendens* 52 implantiert vorliegt.

Das Gefäßprothesensystem 10 in Fig. 4 unterscheidet sich von dem Gefäßprothesensystem 10 in Fig. 3 gezeigt in der, in der Mantelfläche aufweisenden, Fenestrierung bzw. Fenestrierungsbereich 19. Über diese Fenestrierung 19 ist das zweite Lumenende 33 der Seiten-Gefäßprothese 30 ins Lumen 13 der Haupt-Gefäßprothese 12 und über das erste Lumenende 22 der Verankerung-Gefäßprothese 20 in deren Lumen 13 zumindest teilweise einführbar. Eine Fenestrierung 19 kann als präformierte Löcher ausgestaltet sein oder als *in-situ* Fenestrierung. Mittels der Seitengefäßprothese 30, welche durch die Fenestrierung 19 hindurch gesteckt ist, können die Seitengefäße weiterhin mit Blut versorgt werden. Diese Ausgestaltung bietet den Vorteil, dass neben dem Aortenbogen 50 und der *Aorta descendens* 52 auch die *Arteria sublavia* 53 mittels des Gefäßprothesensystems 10 unterstützt wird.

Gemäß einer weiteren Ausführungsform kann also, wie in Fig. 4 gezeigt, auch vorgesehen sein, dass die Haupt-Gefäßprothese aus zwei, miteinander verbindbaren hohlzylindrischen Stentgerüst-Abschnitten 16a, 16b, besteht, derart, dass ein erster Stentgerüst-Abschnitt 16a die Verankerungs-Prothese 20 aufweist, und der zweite Stentgerüst-Abschnitt 16b zur Verlängerung des ersten Stentgerüst-Abschnitts 16a mit diesem verbunden werden kann. Die Seiten-Gefäßprothese 30 kann dann über den zweiten Stentgerüst-Abschnitt 16b in das gemeinsame Lumen und in die im ersten Stentgerüst-Abschnitt 16a vorliegende Verankerungs-Prothese 20 eingeführt werden..

Als Positionierungshilfe während der Implantation und zur Überprüfung der Lage der einzelnen Bestandteile des Gefäßprothesensystems 10, können Röntgenmarker an bestimmten Positionen angebracht werden (nicht gezeigt). Beispielsweise werden derartige Röntgenmarker im Bereich der Lumenenden 14, 15, 22, 23, 32 und/oder 33 oder der Fenestrierung 19 angebracht.

## Patentansprüche

1. Intraluminales Gefäßprothesensystem (10) zur Implantation im Bereich des Aortenbogens (50) eines Patienten, umfassend:
- eine hohlzylindrische Haupt-Gefäßprothese (12), die ein durch die Haupt-Gefäßprothese geführtes Lumen (13), ein erstes Lumenende (14), ein zweites Lumenende (15), ein hohlzylindrisches Stentgerüst (16), ggf. mit einem daran befestigtes Prothesenmaterial (17), eine Länge L1 und einen Durchmesser D1 aufweist, wobei die hohlzylindrische Haupt-Gefäßprothese (12) für die Implantation im Bereich des Aortenbogen (50) und der *Aorta descendens* (52) des Patienten ausgestaltet und dimensioniert ist, und wobei die Haupt-Gefäßprothese (12)
- zumindest eine hohlzylindrische Verankerungs-Gefäßprothese (20) aufweist, welche ein durch die Verankerungs-Gefäßprothese geführtes Lumen (21), ein erstes Lumenende (22) , ein zweites Lumenende (23), ein hohlzylindrisches Stentgerüst (24), ggf. mit einem daran befestigtes Prothesenmaterial (25), eine Länge L2 und einen Durchmesser D2 aufweist, wobei die Verankerungs-Gefäßprothese (20) innerhalb des Lumens (13) der Haupt-Gefäßprothese (12) zumindest über einen Teil der Länge L2 der Verankerungs-Gefäßprothese (20) fest angebracht ist, und wobei der Durchmesser D2 der Verankerungs-Gefäßprothese (20) um mindestens 45% kleiner ist als der Durchmesser D1 der Haupt-Gefäßprothese (12), und wobei die Länge L2 der Verankerungs-Gefäßprothese (20) kleiner ist als die Länge L1 der Haupt-Gefäßprothese (12), wobei das Gefäßprothesensystem (10) ferner
- zumindest eine hohlzylindrische Seiten-Gefäßprothese (30) umfasst, mit einem durch die Seiten-Gefäßprothese (30) geführten Lumen (31), wobei die Seiten-Gefäßprothese (30) ein erstes Lumenende (32), ein zweites Lumenende (33), ein hohlzylindrisches Stentgerüst (34), ggf. mit einem daran befestigtes Prothesenmaterial (35), eine Länge L3 und einen Durchmesser D3 aufweist, und wobei die hohlzylindrische Seiten-Gefäßprothese (30) für die Implantation zur Überbrückung des Abgangs der *Arteria subclavia* (53) und/oder der *Arteria carotis* (54) des Patienten ausgestaltet und dimensioniert ist, derart, dass die Seiten-Gefäßprothese (30) mit ihrem ersten Lumenende (32) in der *Arteria subclavia* (53) und/oder der *Arteria carotis* (54) positionierbar ist, und mit ihrem zweiten Lumenende (33) zur sicheren Verankerung der Seiten-Gefäßprothese (30) über das erste Lumenende (23) der Verankerungs-Gefäßprothese (20) zumindest teilweise in deren Lumen (21) einführbar und darin fixierbar ist, und wobei das zweite Lumenende (33) der Seiten-Gefäßprothese (30) über das erste Lumenende (14) der Haupt-Gefäßprothese (12) in das erste Lumenende (22) der Verankerungs-Gefäßprothese (20) und zumindest teilweise in deren Lumen (21) einführbar ist.

2. Intraluminales Gefäßprothesensystem (10) nach Anspruch 1, **dadurch gekennzeichnet, dass** die Haupt-Gefäßprothese (12) und/oder die Verankerungs-Gefäßprothese (20) und/oder die Seiten-Gefäßprothese (30) ein Stentgerüst (16; 24; 34) sowie ein daran befestigtes Prothesenmaterial (17; 25; 35) aufweist bzw. aufweisen.

3. Intraluminales Gefäßprothesensystem (10) nach Anspruch 1 oder 2, **dadurch gekennzeichnet, dass** das Stentgerüst (16) der Haupt-Gefäßprothese (12) und/oder das Stentgerüst (24) der Verankerungs-Gefäßprothese (20) und/oder das Stentgerüst (34) der Seiten-Gefäßprothese (30) ausgewählt ist aus einem laser-geschnittenen Stentgerüst, einzelnen Stentfedern oder einem geflochtenen Stentgerüst.

4. Intraluminales Gefäßprothesensystem (10) nach einem der Ansprüche 1 bis 3, **dadurch gekennzeichnet, dass** das Stentgerüst (16) der Haupt-Gefäßprothese (12) und/oder das Stentgerüst (24) der Verankerungs-Gefäßprothese (20) und/oder das Stentgerüst (34) der Seiten-Gefäßprothese (30) hintereinander angeordnete, nicht miteinander verbundene mäanderförmige umlaufende Stentringe (18; 26; 36) sowie ein mit den Stentringen (18; 26; 36) fest verbundenes Prothesenmaterial (17; 25; 35) aufweist.

5. Intraluminales Gefäßprothesensystem (10) nach Anspruch 4, **dadurch gekennzeichnet, dass** der mäanderförmgie Umlauf von zumindest einem Stentring (18; 26; 36) des Stentgerüsts (16; 24; 34) eine ungleichmäßige Amplitude aufweist.

6. Intraluminales Gefäßprothesensystem (10) nach einem der Ansprüche 1 bis 5, **dadurch gekennzeichnet, dass** Stentgerüst (16) der Haupt-Gefäßprothese (12) und/oder das Stentgerüst (24) der Verankerungs-Gefäßprothese (20) und/oder das Stentgerüst (34) der Seiten-Gefäßprothese (30) selbstexpandierbar ist.

7. Intraluminales Gefäßprothesensystem (10) nach einem der Ansprüche 1 bis 6, **dadurch gekennzeichnet, dass** der Durchmesser D1 der Haupt-Gefäßprothese (12) von 24 mm bis 42 mm ist, insbesondere 24 mm, 26 mm, 28 mm, 30 mm, 32 mm, 34 mm, 36 mm, 38 mm, 40 mm oder 42 mm.

8. Intraluminales Gefäßprothesensystem (10) nach einem der Ansprüche 1 bis 7, **dadurch gekennzeichnet, dass** der Durchmesser D2 der Verankerung-Gefäßprothese (20) von 6 mm bis 14 mm ist, insbesondere 6 mm, 7 mm, 8 mm, 9 mm, 10 mm, 11 mm, 12 mm, 13 mm oder 14 mm.

9. Intraluminales Gefäßprothesensystem (10) nach einem der Ansprüche 1 bis 8, **dadurch gekennzeichnet, dass** der Durchmesser D3 der Seiten-Gefäßprothese (30) von 6 mm bis 16 mm ist, insbesondere 6 mm, 7 mm, 8 mm, 9 mm, 10 mm, 11 mm, 12 mm, 13 mm, 14 mm, 15 mm oder 16 mm.

10. Intraluminales Gefäßprothesensystem (10) nach einem der Ansprüche 1 bis 9, **dadurch gekennzeichnet, dass** die Verankerungs-Gefäßprothese (20) im Lumen (13) der Haupt-Gefäßprothese (12) an einer Innenwand angebracht, vorzugsweise angenäht, angeklebt, oder angeschweißt ist.

11. Intraluminales Gefäßprothesensystem (10) nach einem der Ansprüche 1 bis 10, **dadurch gekennzeichnet, dass** die Haupt-Gefäßprothese (12) eine Mantelfläche sowie eine in der Mantelfläche vorgesehene Fenestrierung (19) aufweist, über welche das zweite Lumenende (33) der Seiten-Gefäßprothese (30) ins Lumen (13) der Haupt-Gefäßprothese (12) und über das erste Lumenende (22) der Verankerungs-Gefäßprothese (20) in deren Lumen (21) zumindest teilweise einführbar ist.

## Claims

1. An intraluminal vascular prosthesis system (10) for implantation in the region of the aortic arch (50) of a patient, comprising:
- a hollow-cylindrical main vessel prosthesis (12), which has a lumen (13) routed through the main vessel prosthesis, a first lumen end (14), a second lumen end (15), a hollow-cylindrical stent frame (16), optionally with a prosthesis material (17) secured thereon, a length L1 and a diameter D1, wherein the hollow-cylindrical main vessel prosthesis (12) is configured and dimensioned for implantation in the region of the aortic arch (50) and the descending aorta (52) of the patient, and wherein the main vessel prosthesis (12) has
- at least one hollow-cylindrical anchoring vessel prosthesis (20), which has a lumen (21) routed through the anchoring vessel prosthesis, a first lumen end (22), a second lumen end (23), a hollow-cylindrical stent frame (24), optionally with a prosthesis material (25) secured thereon, a length L2 and a diameter D2, wherein the anchoring vessel prosthesis (20) is securely attached within the lumen (13) of the main vessel prosthesis (12), at least over part of the length L2 of the anchoring vessel prosthesis (20), and wherein the diameter D2 of the anchoring vessel prosthesis (20) is at least 45% smaller than the diameter D1 of the main vessel prosthesis (12), and wherein the length L2 of the anchoring vessel prosthesis (20) is shorter than the length L1 of the main vessel prosthesis (12), wherein the vascular prosthesis system (10) moreover comprises
- at least one hollow-cylindrical side vessel prosthesis (30), with a lumen (31) routed through the side vessel prosthesis (30), wherein the side vessel prosthesis (30) has a first lumen end (32), a second lumen end (33), a hollow-cylindrical stent frame (34), optionally with a prosthesis material (35) secured thereon, a length L3 and a diameter D3, and wherein the hollow-cylindrical side vessel prosthesis (30) for implantation is designed and dimensioned to bridge the outlet of the subclavian artery (53) and/or the carotid artery (54) of the patient, in such a way that the side vessel prosthesis (30) can be positioned with its first lumen end (32) in the subclavian artery (53) and/or the carotid artery (54), and, in order to securely anchor the side vessel prosthesis (30), its second lumen end (33) can be inserted at least partially into and fixed in the lumen (21) of the anchoring vessel prosthesis (20) via the first lumen end (22) of the anchoring vessel prosthesis (20), and wherein the second lumen end (33) of the side vessel prosthesis (30) is insertable, via the first lumen end (14) of the main vessel prosthesis (12), into the first lumen end (22) of the anchoring vessel prosthesis (20) and at least partially into the lumen (21) thereof.

2. The intraluminal vascular prosthesis system (10) as claimed in claim 1, **characterized in that** the main vessel prosthesis (12) and/or the anchoring vessel prosthesis (20) and/or the side vessel prosthesis (30) have/has a stent frame (16; 24; 34) and a prosthesis material (17; 25; 35) secured on the latter.

3. The intraluminal vascular prosthesis system (10) as claimed in claim 1 or 2, **characterized in that** the stent frame (16) of the main vessel prosthesis (12) and/or the stent frame (24) of the anchoring vessel prosthesis (20) and/or the stent frame (34) of the side vessel prosthesis (30) is chosen from a laser-cut stent frame, individual stent springs or a braided stent frame.

4. The intraluminal vascular prosthesis system (10) as claimed in one of claims 1 to 3, **characterized in that** the stent frame (16) of the main vessel prosthesis (12) and/or the stent frame (24) of the anchoring vessel prosthesis (20) and/or the stent frame (34) of the side vessel prosthesis (30) has non-interconnected stent rings (18; 26; 36), which are arranged in succession and extend circumferentially in a meandering formation, and a prosthesis material (17; 25; 35) firmly connected to the stent rings (18; 26; 36).

5. The intraluminal vascular prosthesis system (10) as claimed in claim 4, **characterized in that** the meandering circumferential course of at least one stent ring (18; 26; 36) of the stent frame (16; 24; 34) has a non-uniform amplitude.

6. The intraluminal vascular prosthesis system (10) as claimed in one of claims 1 to 5, **characterized in that** the stent frame (16) of the main vessel prosthesis (12) and/or the stent frame (24) of the anchoring vessel prosthesis (20) and/or the stent frame (34) of the side vessel prosthesis (30) is self-expandable.

7. The intraluminal vascular prosthesis system (10) as claimed in one of claims 1 to 6, **characterized in that** the diameter D1 of the main vessel prosthesis (12) is from 24 mm to 42 mm, in particular 24 mm, 26 mm, 28 mm, 30 mm, 32 mm, 34 mm, 36 mm, 38 mm, 40 mm or 42 mm.

8. The intraluminal vascular prosthesis system (10) as claimed in one of claims 1 to 7, **characterized in that** the diameter D2 of the anchoring vessel prosthesis (20) is from 6 mm to 14 mm, in particular 6 mm, 7 mm, 8 mm, 9 mm, 10 mm, 11 mm, 12 mm, 13 mm or 14 mm.

9. The intraluminal vascular prosthesis system (10) as claimed in one of claims 1 to 8, **characterized in that** the diameter D3 of the side vessel prosthesis (30) is from 6 mm to 16 mm, in particular 6 mm, 7 mm, 8 mm, 9 mm, 10 mm, 11 mm, 12 mm, 13 mm, 14 mm, 15 mm or 16 mm.

10. The intraluminal vascular prosthesis system (10) as claimed in one of claims 1 to 9, **characterized in that** the anchoring vessel prosthesis (20) in the lumen (13) of the main vessel prosthesis (12) is mounted on an inner wall, preferably by sewing, adhesive bonding or welding.

11. The intraluminal vascular prosthesis system (10) as claimed in one of claims 1 to 10, **characterized in that** the main vessel prosthesis (12) has a jacket surface, and a fenestration (19) which is provided in the jacket surface and via which the second lumen end (33) of the side vessel prosthesis (30) is insertable into the lumen (13) of the main vessel prosthesis (12) and at least partially into the lumen (21) of the anchoring vessel prosthesis (20) via the first lumen end (22) of the latter.

## Revendications

1. Système de prothèse vasculaire intraluminale (10) destiné à être implanté dans la région de l'arc aortique (50) d'un patient, comprenant :
- une prothèse vasculaire principale (12) cylindrique creuse qui présente une lumière (13) guidée à travers la prothèse vasculaire principale, une première extrémité de lumière (14), une seconde extrémité de lumière (15), une armature d'endoprothèse (16) cylindrique creuse, le cas échéant comportant un matériau prothétique (17) fixé à celle-ci, une longueur L1 et un diamètre D1, dans lequel la prothèse vasculaire principale (12) cylindrique creuse est conçue et dimensionnée pour l'implantation dans la région de l'arc aortique (50) et de l'aorte descendante (52) du patient, et dans lequel la prothèse vasculaire principale (12)
- présente au moins une prothèse vasculaire d'ancrage (20) cylindrique creuse qui présente une lumière (21) guidée à travers la prothèse vasculaire d'ancrage, une première extrémité de lumière (22), une seconde extrémité de lumière (23), une armature d'endoprothèse (24) cylindrique creuse, le cas échéant comportant un matériau prothétique (25) fixé à celle-ci, une longueur L2 et un diamètre D2, dans lequel la prothèse vasculaire d'ancrage (20) est montée de manière fixe à l'intérieur de la lumière (13) de la prothèse vasculaire principale (12) au moins sur une partie de la longueur L2 de la prothèse vasculaire d'ancrage (20), et dans lequel le diamètre D2 de la prothèse vasculaire d'ancrage (20) est inférieur d'au moins 45 % au diamètre D1 de la prothèse vasculaire principale (12), et dans lequel la longueur L2 de la prothèse vasculaire d'ancrage (20) est inférieure à la longueur L1 de la prothèse vasculaire principale (12), dans lequel le système de prothèse vasculaire (10)
- comprend en outre au moins une prothèse vasculaire latérale (30) cylindrique creuse, comportant une lumière (31) guidée à travers la prothèse vasculaire latérale (30), dans lequel la prothèse vasculaire latérale (30) présente une première extrémité de lumière (32), une seconde extrémité de lumière (33), une armature d'endoprothèse (34) cylindrique creuse, le cas échéant comportant un matériau prothétique (35) fixé à celle-ci, une longueur L3 et un diamètre D3, et dans lequel la prothèse vasculaire latérale (30) cylindrique creuse est conçue et dimensionnée pour l'implantation afin de ponter la sortie de l'artère subclavière (53) et/ou de l'artère carotide (54) du patient, de telle sorte que la prothèse vasculaire latérale (30) peut être positionnée avec sa première extrémité de lumière (32) dans l'artère subclavière (53) et/ou dans l'artère carotide (54), et, pour l'ancrage sûr de la prothèse vasculaire latérale (30), peut être introduite avec sa seconde extrémité de lumière (33) au moins partiellement dans la lumière (21) de la prothèse vasculaire d'ancrage (20) par l'intermédiaire de la première extrémité de lumière (23) de celle-ci et peut y être fixée, et dans lequel la seconde extrémité de lumière (33) de la prothèse vasculaire latérale (30) peut être introduite, par l'intermédiaire de la première extrémité de lumière (14) de la prothèse vasculaire principale (12), dans la première extrémité de lumière (22) de la prothèse vasculaire d'ancrage (20) et au moins partiellement dans la lumière (21) de celle-ci.

2. Système de prothèse vasculaire intraluminale (10) selon la revendication 1, **caractérisé en ce que** la prothèse vasculaire principale (12) et/ou la prothèse vasculaire d'ancrage (20) et/ou la prothèse vasculaire latérale (30) présentent une armature d'endoprothèse (16 ; 24 ; 34) ainsi qu'un matériau prothétique (17 ; 25 ; 35) fixé à celle-ci.

3. Système de prothèse vasculaire intraluminale (10) selon la revendication 1 ou 2, **caractérisé en ce que** l'armature d'endoprothèse (16) de la prothèse vasculaire principale (12) et/ou l'armature d'endoprothèse (24) de la prothèse vasculaire d'ancrage (20) et/ou l'armature d'endoprothèse (34) de la prothèse vasculaire latérale (30) sont choisies parmi une armature d'endoprothèse découpée au laser, des ressorts d'endoprothèse individuels ou une armature d'endoprothèse tressée.

4. Système de prothèse vasculaire intraluminale (10) selon l'une des revendications 1 à 3, **caractérisé en ce que** l'armature d'endoprothèse (16) de la prothèse vasculaire principale (12) et/ou l'armature d'endoprothèse (24) de la prothèse vasculaire d'ancrage (20) et/ou l'armature d'endoprothèse (34) de la prothèse vasculaire latérale (30) présentent des anneaux d'endoprothèse (18 ; 26 ; 36) périphériques en forme de méandres disposés les uns derrière les autres et non reliés les uns aux autres, ainsi qu'un matériau prothétique (17 ; 25 ; 35) relié de manière fixe aux anneaux d'endoprothèse (18 ; 26 ; 36).

5. Système de prothèse vasculaire intraluminale (10) selon la revendication 4, **caractérisé en ce que** la périphérie en forme de méandres d'au moins un anneau d'endoprothèse (18 ; 26 ; 36) de l'armature d'endoprothèse (16 ; 24 ; 34) présente une amplitude non uniforme.

6. Système de prothèse vasculaire intraluminale (10) selon l'une des revendications 1 à 5, **caractérisé en ce que** l'armature d'endoprothèse (16) de la prothèse vasculaire principale (12) et/ou l'armature d'endoprothèse (24) de la prothèse vasculaire d'ancrage (20) et/ou l'armature d'endoprothèse (34) de la prothèse vasculaire latérale (30) sont auto-expansibles.

7. Système de prothèse vasculaire intraluminale (10) selon l'une des revendications 1 à 6, **caractérisé en ce que** le diamètre D1 de la prothèse vasculaire principale (12) va de 24 mm à 42 mm, en particulier est de 24 mm, 26 mm, 28 mm, 30 mm, 32 mm, 34 mm, 36 mm, 38 mm, 40 mm ou 42 mm.

8. Système de prothèse vasculaire intraluminale (10) selon l'une des revendications 1 à 7, **caractérisé en ce que** le diamètre D2 de la prothèse vasculaire d'ancrage (20) va de 6 mm à 14 mm, en particulier est de 6 mm, 7 mm, 8 mm, 9 mm, 10 mm, 11 mm, 12 mm, 13 mm ou 14 mm.

9. Système de prothèse vasculaire intraluminale (10) selon l'une des revendications 1 à 8, **caractérisé en ce que** le diamètre D3 de la prothèse vasculaire latérale (30) va de 6 mm à 16 mm, en particulier est de 6 mm, 7 mm, 8 mm, 9 mm, 10 mm, 11 mm, 12 mm, 13 mm, 14 mm, 15 mm ou 16 mm.

10. Système de prothèse vasculaire intraluminale (10) selon l'une des revendications 1 à 9, **caractérisé en ce que** la prothèse vasculaire d'ancrage (20) est montée, de préférence cousue, collée ou soudée, dans la lumière (13) de la prothèse vasculaire principale (12) sur une paroi interne.

11. Système de prothèse vasculaire intraluminale (10) selon l'une des revendications 1 à 10, **caractérisé en ce que** la prothèse vasculaire principale (12) présente une surface d'enveloppe ainsi qu'une fenestration (19) prévue dans la surface d'enveloppe, par l'intermédiaire de laquelle fenestration la seconde extrémité de lumière (33) de la prothèse vasculaire latérale (30) peut être introduite au moins partiellement dans la lumière (13) de la prothèse vasculaire principale (12) et peut être introduite au moins partiellement dans la lumière (21) de la prothèse vasculaire d'ancrage (20) par l'intermédiaire de la première extrémité de lumière (22) de celle-ci.
